# EUROPÄISCHE PATENTSCHRIFT

(11) **EP 0 774 119 B1**
(45) Veröffentlichungstag und Bekanntmachung des Hinweises auf die Patenterteilung: **03.03.2004**
(21) Anmeldenummer: 95927713.8
(22) Anmeldetag: 24.07.1995
(51) Int. Cl.: G01N 33/532, G01N 33/533, G01N 33/58, C12Q 1/68, G01N 33/543

(54) **OLIGOMERE TRÄGERMOLEKÜLE MIT DEFINIERT EINGEBAUTEN MARKIERUNGSGRUPPEN UND HAPTENEN**
OLIGOMER CARRIER MOLECULES IN WHICH MARKER GROUPS AND HAPTENS ARE SELECTIVELY INCORPORATED
MOLECULES PORTEUSES OLIGOMERES DANS LESQUELLES SONT INCORPORES SELECTIVEMENT DES GROUPES MARQUEURS ET DES HAPTENES

(30) Priorität: 25.07.1994 DE 4426276; 31.08.1994 DE 4430998; 31.08.1994 DE 4430973; 04.11.1994 DE 4439345
(43) Veröffentlichungstag der Anmeldung: 21.05.1997
(73) Patentinhaber: Roche Diagnostics GmbH, 68305 Mannheim (DE)
(72) Erfinder: JOSEL, Hans-Peter, D-82362 Weilheim (DE); FINKE, Andreas, D-82377 Penzberg (DE); HERRMANN, Rupert, D-82362 Weilheim (DE); HÖSS, Eva, D-82319 Starnberg (DE); MARSCHALL, Andreas, D-69469 Weinheim (DE); SEIDEL, Christoph, D-82362 Weilheim (DE)
(74) Vertreter: Weiss, Wolfgang, Dipl.-Chem. Dr.
(86) Internationale Anmeldenummer: PCT/EP1995/002915
(87) Internationale Veröffentlichungsnummer: WO 1996/003650

(56) Entgegenhaltungen:
- EP-A- 0 135 071
- EP-A- 0 154 884
- EP-A- 0 155 224
- EP-A- 0 178 450
- EP-A- 0 507 587
- WO-A-91/16344
- ANALYTICAL BIOCHEMISTRY, Bd. 193, Nr. 2, März 1991 NEW YORK US, Seiten 272-279, R. BREDEHORST ET AL. 'Novel trifunctional carrier molecule for the fluorescent labeling of haptens.'

## Beschreibung

Die vorliegende Erfindung betrifft Konjugate, Verfahren zu deren Herstellung sowie die Verwendung dieser Konjugate als Antigene in immunologischen Nachweisverfahren oder zur DNA-Diagnostik.

Der Nachweis von Immunglobulinen in Körperflüssigkeiten, insbesondere in Humanseren, wird zur Diagnostik von Infektionen mit Mikroorganismen, insbesondere Viren, wie etwa HIV, Hepatitis-Viren, etc. verwendet. Das Vorhandensein von spezifischen Immunglobulinen in der untersuchten Probe wird üblicherweise durch Reaktion mit einem oder mehreren Antigenen, die mit den spezifischen Immunglobulinen reagieren, nachgewiesen. Verfahren zur Bestimmung von spezifischen Immunglobulinen in der Probeflüssigkeit müssen sensitiv, zuverlässig, einfach und schnell sein.

Ein weiteres immunologisches Verfahren ist der kompetitive Immunoassay, bei dem der qualitative und quantitative Nachweis eines Analyten dadurch erfolgt, daß ein mit dem Analyten immunologisch analoges Hapten und der Analyt um Bindungsstellen auf einem Rezeptor, z.B. einem Antikörper konkurrieren. Das Analytenanalogon wird dabei im allgemeinen in markierter oder an eine Festphase bindefähiger Form eingesetzt.

In den letzten Jahren wurden zunehmend Nachweissysteme auf Basis nicht-radioaktiver Markierungsgruppen entwickelt, bei denen das Vorhandensein eines Analyten, z.B. eines spezifischen Antikörpers, in der untersuchten Probe mit Hilfe optischer (z.B. Lumineszenz oder Fluoreszenz), NMR-aktiver oder Metall-präzipitierender Detektionssysteme bestimmt werden konnte.

EP-A-0 307 149 offenbart einen Immuntest für einen Antikörper, bei dem zwei rekombinante Polypeptide als Antigene verwendet werden, von denen eines an einer festen Phase immobilisiert ist, und das andere eine Markierungsgruppe trägt, wobei beide rekombinanten Antigene in unterschiedlichen Organismen exprimiert werden, um die Spezifität des Nachweises zu erhöhen.

EP-A-0 366 673 offenbart ein Verfahren zum Nachweis von Antikörpern in einer Probe, bei dem ein Antikörper durch Reaktion mit einem gereinigten, markierten Antigen und dem gleichen gereinigten Antigen in einer Festphasen-gebundenen Form nachgewiesen wird. Als Antigen wird beispielsweise humanes IgG offenbart.

EP-A-0 386 713 beschreibt ein Verfahren zum Nachweis von Antikörpern gegen HIV unter Verwendung von zwei festen Trägern, wobei an beide festen Träger verschiedene HIV-Antigene immobilisiert werden, die jeweils mit einem Aliquot einer Probe und einem markierten HIV-Antigen in Kontakt gebracht werden, wobei das Vorhandensein von Antikörpern durch eine positive Reaktion in mindestens einem der Tests nachgewiesen wird. Als HIV-Antigene werden rekombinant hergestellte Polypeptide offenbart.

EP-A-0 507 586 beschreibt ein Verfahren zur Durchführung eines immunologischen Tests für ein spezifisches Immunglobulin, bei dem eine Probe mit zwei zur Bindung des Immunglobulins fähigen Antigenen in Kontakt gebracht wird, wobei das erste Antigen eine zur Bindung an einen festen Träger geeignete Gruppe trägt, und das zweite Antigen eine Markierungsgruppe trägt. Die Markierungsgruppe kann eine direkte Markierungsgruppe sein, z.B. ein Enzym, ein Chromogen, ein Metallteilchen, oder auch eine indirekte Markierungsgruppe, d.h. die am Antigen angebrachte Markierungsgruppe kann mit einem Rezeptor für die Markierungsgruppe, der wiederum eine signalerzeugende Gruppe trägt, reagieren. Als Beispiel für eine solche indirekte Markierungsgruppe wird ein Fluoresceinderivat genannt, dessen Rezeptor ein Antikörper ist, der wiederum mit einem Enzym gekoppelt ist. Als Antigene werden Polypeptide, wie etwa das Hepatitis B-Oberflächenantigen offenbart. In dieses Antigen werden durch Derivatisierung SH-Gruppen eingeführt, mit denen das Fluorescein gekoppelt wird.

EP-A-0 507 587 offenbart ein spezifisch zum Nachweis von IgM Antikörpern geeignetes Verfahren, bei dem die Probe mit einem markierten Antigen, das gegen den nachzuweisenden Antikörper gerichtet ist, und einem zweiten Antikörper, der ebenfalls gegen den nachzuweisenden Antikörper gerichtet und an eine Festphase bindefähig ist, inkubiert wird.

EP-A-0 199 804 und EP-A-0 580 979 offenbaren ein immunologisches Nachweisverfahren unter Verwendung von Antigenen, die mit lumineszierenden Metallchelatgruppen, insbesondere mit Rutheniumund Osmiumchelatgruppen markiert sind. Als Antigene werden Immunglobuline verwendet, die durch Reaktion mit aktivierten Metallkomplexen statistisch markiert werden.

EP-A-0 178 450 offenbart Metallchelate, insbesondere Rutheniumkomplexe, an die ein immunologisch aktives Material, beispielsweise Antikörper, gekoppelt werden kann. Die Kopplung erfolgt durch statistische Reaktion des immunologisch reaktiven Materials mit dem Metallchelat.

EP-A-0 255 534 offenbart einen Lumineszenz-Immunoassay unter Verwendung eines Metallchelat-gekoppelten Antigens oder Antikörpers. Die Kopplung erfolgt beispielsweise durch statistische Reaktion eines Metallchelat-Aktivesterderivats mit einem Antikörper.

WO 90/05301 offenbart ein Verfahren zum Nachweis und zur quantitativen Bestimmung von Analyten durch Elektrochemilumineszenz unter Verwendung von lumineszierenden Metallchelaten, die an (i) einen zugesetzten Analyten, (ii) einen Bindepartner des Analyten oder (iii) eine reaktive Komponente, die mit (i) oder (ii) binden kann, gekoppelt sind. Die Lumineszenzmessung erfolgt nach Bindung der Metallchelate an aktivierte und gegebenenfalls magnetische Mikropartikel.

Bei den aus dem Stand der Technik bekannten immunologischen Nachweisverfahren für Antikörper werden üblicherweise Polypeptid-Antigene verwendet, die meist durch rekombinante DNA-Methoden erzeugt wurden. Beim Einsatz derartiger Polypeptid-Antigene können jedoch Probleme auftreten. So können rekombinante Polypeptide oft nur in Form von Fusionspolypeptiden erzeugt werden, bei denen der Fusionsanteil zu falsch positiven Resultaten im Test führen kann. Weiterhin zeigen durch rekombinante Expression erzeugte Polypeptide oft eine nur geringe Stabilität in der Probelösung und neigen zu Aggregation. Ein weiterer Nachteil ist, daß oft keine selektive und reproduzierbare Einführung von Markierungsgruppen in solche Polypeptide möglich ist.

Überdies ist die Herstellung rekombinanter Polypeptidantigene mit hohen Kosten verbunden und es können größere Schwankungen in der immunologischen Reaktivität bei verschiedenen Chargen rekombinanter Polypeptide auftreten.

Auch bei kompetitiven Immunoassays, die sehr hohe Anforderungen an die Sensitivität und Präzision stellen, können beim Nachweis von nur in sehr geringen Konzentrationen vorliegenden Analyten wie etwa Estradiol oder Testosteron, insbesondere bei Nachweissystemen auf Basis von Elektrochemilumineszenz die geforderten unteren Nachweisgrenzen unter Verwendung bekannter Antigene nur sehr schwer erreicht werden.

EP-A-0 135 071 beschreibt einen Lumineszenz-Immunoassay für Haptene, bestehend aus einem für das jeweilige Hapten spezifischen Antikörper und einem chemilumineszent markierten Haptenkonjugat. Die gemäß den Beispielen von EP-A-0 135 071 als Träger verwendeten Polypeptide weisen wesentlich mehr als 100 Aminosäuren auf, die eine sehr große Anzahl von funktionellen Gruppen enthalten. Dies hat zur Folge, daß die Einführung der Haptene bzw. Markierungsgruppen nur statistisch erfolgen kann.

Bredehorst et al., (Anal. Biochem. 193 (1991), 272-279) offenbart Konjugate mit der 21 Aminosäuren langen Insulin-A-Kette als polymerer Träger.

An den polymeren Träger sind ein Haptenmolekul an den N-Terminus und drei Fluoreszenzmarkierungsgruppen an Glutaminsäurereste innerhalb der Trägersequenz gekoppelt. Die Thiolgruppen innerhalb der Trägersequenz sind zu S-Sulfonatgruppen derivatisiert. Derartige Konjugate sind auch in WO 91/16344 beschrieben.

EP-A-0 155 224 beschreibt Konjugate aus einem hochmolekularen Träger, an den Haptenmoleküle und Markierungsgruppen gekoppelt sind.

Das der vorliegenden Erfindung zugrundeliegende Problem war somit die Bereitstellung eines Verfahrens, bei dem auf einfache und effiziente Weise Antigene für immunologische Tests erzeugt werden können, wobei die Nachteile der aus dem Stand der Technik bekannten Antigene mindestens teilweise beseitigt werden. Weiterhin soll das Verfahren eine selektive und reproduzierbare Einführung von Markierungsgruppen in die Antigene ermöglichen.

Das der Erfindung zugrundliegende Problem wird gelöst durch Konjugate nach Anspruch 1 und ein Verfahren zur Herstellung von Konjugaten nach Anspruch 10.

Die Erfindung betrifft ein Konjugat, umfassend einen polymeren Träger mit maximal 100 monomeren Einheiten, der gekoppelt an reaktive Gruppen 1 bis 10 Haptenmoleküle und 1 bis 10 Markierungs- oder Festphasenbindungsgruppen enthält, wobei die monomeren Einheiten aus Aminosäuren ausgewählt sind, und wobei die Haptenmoleküle und die Harkierungs- oder Festphasenbindungsgruppen über die primären Amino- oder/und Thiolgruppen der Aminosäuremonomere an den polymeren Träger gekoppelt sind.

Durch Verwendung der erfindungsgemäßen Konjugate, die 1-10 Haptenmoleküle und eine definierte Anzahl von Markierungs- oder Festphasenbindungsgruppen enthalten, als Antigene in einem immunologischen Nachweisverfahren kann überraschenderweise eine wesentlich höhere Sensitivität und Präzision bei einer gleichzeitig verringerten unteren Nachweisgrenze gegenüber bekannten monomeren und multimeren Antigenen erreicht werden. Darüber hinaus lassen sich die erfindungsgemäßen Konjugate in sehr einfacher Weise durch Festphasensynthese, z.B. eine Peptid-Festphasensynthese, aufbauen. Hierzu können an jeweils vorbestimmten Positionen monomere Einheiten, z.B. Aminosäurederivate, eingebaut werden, die mit einem Haptenmolekül oder einer Markierungs- bzw. Festphasenbindungsgruppe derivatisiert sind. Überdies kann an Positionen der Trägerkette, an denen sich Monomere mit freien funktionellen Gruppen befinden, nach Abschluß der Festphasensynthese ein zusätzlicher selektiver Einbau von Haptenen oder Markierungs-bzw. Festphasenbindungsgruppen erfolgen. Dadurch ist ein definierter und reproduzierbarer Einbau von Haptenmolekülen und Markierungs- bzw. Festphasenbindungsgruppen in das Konjugat möglich. Die Abstände zwischen den einzelnen Gruppen auf dem Konjugat können genau festgelegt und gegebenenfalls variiert werden. Durch Auswahl des Abstandes von Markierungsgruppen auf dem Konjugat kann das Signalquenching gering gehalten werden, so daß die Signalstärke proportional mit der Zahl von Markierungsgruppen zunimmt. Auch eine festgelegte räumliche Orientierung von Markierungsgruppen trägt, z.B. bei helikalen Trägern, zur Verbesserung der Signalstärke bei. Vorzugsweise betragen die Abstände zwischen Markierungsgruppen daher 3-6 oder/und 13-16 monomere Einheiten bei helikalen Trägern, z.B. einzel- oder insbesondere doppelsträngige Nukleinsäuren.

Das polymere Trägermolekül, das das Rückgrat des Konjugats bildet, hat eine Länge von maximal 100 monomeren Einheiten, vorzugsweise von 3 - 80 monomeren Einheiten und besonders bevorzugt von 5 - 60 monomeren Einheiten.

Die monomeren Einheiten werden aus Aminosäuren, Nukleotiden, Nukleotidanaloga und peptidischen Nukleinsäuren ausgewählt. Vorzugsweise umfaßt der polymere Träger eine aus Aminosäuren aufgebaute Peptidkette, vorzugsweise eine lineare Peptidkette. Der Träger kann jedoch auch eine aus Nukleotiden oder Nukleotidanaloga aufgebaute Nukleinsäurekette umfassen, an deren reaktiven Seitengruppen Haptenmoleküle und Markierungs- bzw. Festphasenbindungsgruppen angekoppelt sind. Diese Nukleinsäurekette kann als Einzel- oder als Doppelstrang vorliegen. Bei doppelsträngigen Nukleinsäureträgern findet man oft eine Erhöhung der Signalstärke von Markierungsgruppen, z.B. eine verbesserte Quantenausbeute bei Fluoreszenzmarkierungsgruppen.

Weiterhin kann der polymere Träger aus peptidischen Nukleinsäuren (PNA) aufgebaut sein. Peptidische Nukleinsäuren umfassen ein Polyamidrückgrat aus gleichen oder verschiedenen monomeren Einheiten der Formel

(CH₂)ₖ-CHR'-N[CO-(CH₂)ᵢ-L]-CH₂-(CH₂)ₘ-NH-CO-,

wobei L aus der Gruppe, bestehend aus Wasserstoff, Phenyl, natürlich vorkommenden Nukleobasen und nicht natürlich vorkommenden Nukleobasen ausgewählt ist, R' aus der Gruppe, bestehend aus Wasserstoff und den Seitenketten natürlich oder nicht natürlich vorkommender Aminosäuren, vorzugsweise α-Aminosäuren ausgewählt ist, k und m jeweils unabhängig 0 oder 1 sind und i unabhängig von 0 bis 5 ist. Die Haptenmoleküle und Markierungs- bzw. Festphasenbindungsgruppen können an die Nukleobasen- oder/und Aminosäureseitenketten der peptidischen Nukleinsäure gekoppelt sein. Peptidische Nukleinsäuren und ihre Herstellung sind in WO92/20703 beschrieben. Auf diese Offenbarung wird hiermit Bezug genommen.

Auch bei peptidischen Nukleinsäuren kann der Träger als Einzeloder Doppelstrang vorliegen. Besonders bevorzugt sind doppelsträngige Träger mit mindestens einem PNA-Strang, z.B. einem PNA-Strang und einem Nukleinsäurestrang, z.B. einem DNA-Strang.

Das Konjugat enthält 1-10 Haptenmoleküle, vorzugsweise 1-6 Haptenmoleküle und besonders bevorzugt 1 oder 2 Haptenmoleküle. Das Hapten ist vorzugsweise ein immunologisch reaktives Molekül mit einer Molekularmasse von 100-2000 Da. Derartige Haptene können z.B. aus pharmakologischen Wirkstoffen, wie etwa Antibiotika, Opiaten, Amphetaminen, Barbituraten, Cytostatika (z.B. Gentamicin, Tobramycin, Vancomycin etc.), Paracetamol, Salicylaten, Phenytoin, Chinin und Chininderivaten, Theophyllin etc., Hormonen und Metaboliten wie etwa Sterinen, Gallensäuren, Sexualhormonen (z.B. Estradiol, Estriol, Testosteron, Progesteron, Pregnenolon und Derivate davon), Corticoiden (z.B. Cortisol, Corticosteron, Cortison und Derivate davon), Cardenoliden und Cardenolid-Glycosiden (z.B. Digoxin, Digoxigenin, Strophantin, Bufadienolide etc.) Steroid-Sapogeninen, Steroidalkaloiden, Peptidhormonen, Creatinin, Schilddrüsenhormonen (z.B. T₃, T₄), Neurotransmittern (z.B. Serotonin, Cholin, γ-Aminobuttersäure), Vitaminen und Mediatoren wie etwa Prostaglandinen, Leukotrienen, Leuko-En-diinen und Thromboxanen ausgewählt werden.

Andererseits kann das Hapten auch aus immunologisch reaktiven Peptidepitopen ausgewählt werden, die eine Länge von vorzugsweise bis zu 30 Aminosäuren besitzen. Derartige Peptidepitope können beispielsweise aus pathogenen Organismen, z.B. Bakterien, Viren und Protozoen oder aus Autoimmun-Antigenen, stammen. Beispielsweise können die immunologisch reaktiven Peptidepitope aus viralen Antigenen, z.B. den Aminosäuresequenzen von HIV I, HIV II oder Hepatitis C-Virus (HCV), stammen.

Außerdem kann das Hapten auch aus Nukleinsäuren mit einer Länge von vorzugsweise bis zu 50 Nukleotiden ausgewählt sein, die zu einer Nukleinsäuresequenz, die in der Probe nachgewiesen werden soll, komplementär sind. Schließlich kann das Hapten auch aus peptidischen Nukleinsäuren mit einer Länge von bis zu 50 Monomereinheiten ausgewählt sein.

Weiterhin enthält das erfindungsgemäße Konjugat 1-10, vorzugsweise 2-8 Markierungs- oder Festphasenbindungsgruppen. Bevorzugte Beispiele für Markierungsgruppen sind lumineszierende Metallchelate und Fluoreszenzmarkierungen. Bevorzugte Beispiele für Festphasenbindungsgruppen sind Biotin und Biotinanaloga, wie etwa Desthiobiotin und Imminobiotin, die eine spezifische Reaktion mit Streptavidin oder Avidin eingehen können.

Die Haptenmoleküle und Markierungs- bzw. Festphasenbindungsgruppen sind über primäre Amino- oder/und Thiol-gruppen an die Trägerkette gekoppelt. Derartige Gruppen können durch Einbau von entsprechenden Monomeren, z.B. Aminosäuren wie etwa Lysin, Ornithin, Hydroxylysin oder Cystein, in die Trägerkette erzeugt werden.

In bestimmten Ausführungsformen der vorliegenden Erfindung kann es bevorzugt sein, wenn ein Spacer zwischen das Hapten bzw. die Markierungs- oder Festphasenbindungsgruppe und die Trägerkette eingebaut wird. Der Spacer ist vorzugsweise flexibel und hat eine Kettenlänge von vorzugsweise 3-30 Atomen. Besonders bevorzugt enthält der Spacer hydrophile Gruppen, z.B. Oxyalkylen- oder/und Hydroxy-Seitengruppen.

Bevorzugte Markierungsgruppen sind lumineszierende Metallchelate, d.h. Metallchelate, die eine nachweisbare Lumineszenzreaktion erzeugen. Der Nachweis dieser Lumineszenzreaktion kann beispielsweise durch Fluoreszenz- oder durch Elektrochemilumineszenzmessung erfolgen. Das Metall dieser Metallchelate ist beispielsweise ein Übergangsmetall oder ein Seltenerdenmetall. Vorzugsweise ist das Metall Ruthenium, Osmium, Rhenium, Iridium, Rhodium, Platin, Indium, Palladium, Molybdän, Techneticum, Kupfer, Chrom oder Wolfram. Besonders bevorzugt sind Ruthenium, Iridium, Rhenium, Chrom und Osmium. Am meisten bevorzugt ist Ruthenium.

Die Liganden, die zusammen mit dem Metall das Metallchelat bilden, sind üblicherweise Polydentat-Liganden, d.h. Liganden mit mehreren Koordinationsstellen. Polydentat-Liganden umfassen beispielsweise aromatische und aliphatische Liganden. Geeignete aromatische Polydentat-Liganden beinhalten aromatische heterocyclische Liganden. Bevorzugte aromatische heterocyclische Liganden sind N-haltige Polyheterocyclen wie etwa z.B. Bipyridyl, Bipyrazyl, Terpyridyl und Phenanthrolyl. Diese Liganden können beispielsweise Substituenten wie etwa Alkyl, substituiertes Alkyl, Aryl, substituiertes Aryl, Aralkyl, Carboxylat, Carboxyaldehyd, Carboxamid, Cyano, Amino, Hydroxy, Imino, Hydroxycarbonyl, Aminocarbonyl, Amidin, Guanidinium, Ureid, schwefelhaltige Gruppen, phosphorhaltige Gruppen und die Carboxylatester von N-Hydroxysuccinimid umfassen. Bevorzugte Liganden enthalten C₂-C₃-Alkylenoxy-, C₂-C₃-Alkylenthio- und C₂-C₃-Alkylenaminogruppen, insbesondere Ethylenoxygruppen. Das Chelat kann auch einen oder mehrere Monodentat-Liganden enthalten. Beispiele von Monodentat-Liganden umfassen Kohlenmonoxid, Cyanide, Isocyanide, Halogenide und aliphatische, aromatische und heterocyclische Phosphine, Amine, Stilbene und Arsine.

Besonders bevorzugt wird das lumineszierende Metallchelat ausgewählt aus Metallchelaten mit Bipyridyl- oder Phenanthrolyl-Liganden. Beispiele geeigneter Metallchelate und ihre Herstellung sind in EP-A-0 178 450, EP-A-0 255 534, EP-A-0 580 979 und WO 90/05301 beschrieben. Auf diese Offenbarung wird hiermit Bezug genommen. Am meisten bevorzugte Metallchelate sind Ruthenium-(bipyridyl)₃-Chelate. Diese Chelate sind in Form von Aktivesterderivaten kommerziell erhältlich, z.B. von Igen Inc. (Rockville, MD, USA).

Bei Verwendung eines lumineszierenden Metallkomplexes, der durch eine Elektrochemilumineszenz-Reaktion nachweisbar ist, als Markierungsgruppe hat sich der Einbau von mindestens einem positiven oder/und negativen Ladungsträger, z.B. Amino- oder Carboxylatgruppen, in die Trägerkette oder/und in den Spacer zwischen Metallkomplex und Trägerkette als günstig erwiesen. Besonders bevorzugt enthält die Trägerkette eine oder mehrere negative Ladungen, die z.B. durch Einbau von Glutaminsäure oder Asparaginsäure während der Synthese erzeugt werden können. Auch bei anderen Markierungs- bzw. Festphasenbindungsgruppen, z.B. Fluoreszenzgruppen oder Biotin, kann der Einbau von Ladungsträgern in die Trägerkette oder/und in einen Spacer bevorzugt sein.

Ein weiteres Beispiel für bevorzugte Markierungsgruppen sind Fluoreszenzmarkierungen, wie etwa Fluorescein, Cumarin, Rhodamin, Resorufin, Cyanin und Derivate davon. Bei Verwendung von fluoreszierenden Markierungsgruppen hat sich eine helikale Struktur des Trägerrückgrats als günstig erwiesen, welche die Fluoreszenzmarkierungsgruppen hinsichtlich Raumorientierung und Abstand fixiert, um eine Fluoreszenzlöschung durch einen Energietransfer zu verhindern. Beispiele für Monomere, die eine geeignete helikale Struktur ergeben, sind Prolin oder ein peptidisches Nukleinsäurederivat mit einer Prolinseitengruppe.

Die Herstellung der erfindungsgemäßen Konjugate, umfassend einen polymeren Träger mit maximal 100 monomeren Einheiten, erfolgt durch ein Verfahren, bei dem man einen polymeren Träger, vorzugsweise einen Peptidträger aus monomeren Einheiten an einer Festphase synthetisiert, wobei man (a) während der Synthese an vorbestimmten Positionen des Trägers Monomerderivate einführt, die kovalent mit Haptenmolekülen oder/und Markierungs- bzw. Festphasenbindungsgruppen gekoppelt sind, oder/und (b) nach der Synthese aktivierte Haptenmoleküle oder/und Markierungs- bzw. Festphasenbindungsgruppen an reaktive Gruppen des Trägers koppelt, wobei in Variante (b) die Kopplung an die primären Amino- oder/und Thiol-Gruppen der Aminosäuremonomere nach Abspaltung von Schutzgruppen der zur Festphasensynthese verwendeten Monomerderivate erfolgt.

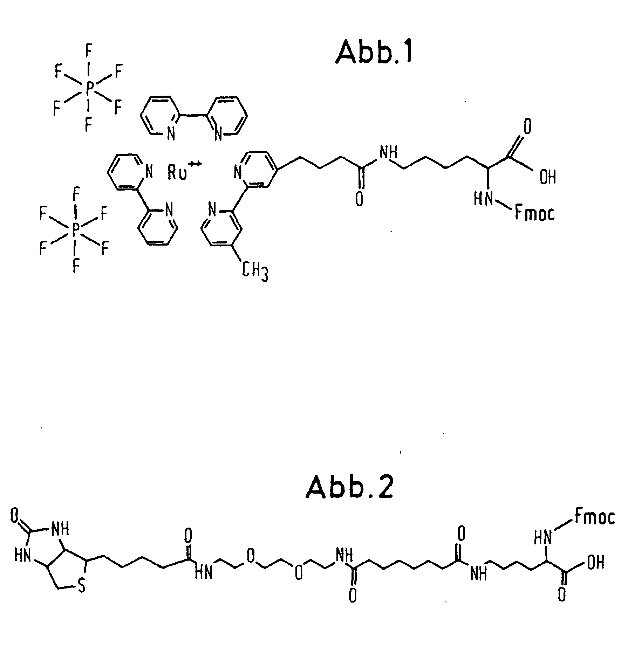

Bei Variante (a) des erfindungsgemäßen Verfahrens wird während der Festphasensynthese ein Monomerderivat eingeführt, das kovalent, vorzugsweise über eine primäre Aminoseitengruppe einer basischen Aminosäure wie Lysin oder Ornithin oder über eine Thiolseitengruppe einer Aminosäure wie Cystein mit einem Haptenmolekül oder/und einer Markierungs- bzw. Festphasenbindungsgruppe gekoppelt ist. Die Herstellung entsprechender Monomerderivate kann beispielsweise durch Kopplung eines aktivierten Haptenmoleküls oder einer aktivierten Markierungs-bzw. Festphasenbindungsgruppe, z.B. eines Aktivesterderivats an eine freie primäre Aminogruppe oder eines Maleimidderivats an eine freie Thiolgruppe von gegebenenfalls partiell geschützten Monomerderivaten, z.B. Aminosäurederivaten, erfolgen. In Abb. 1 ist ein bevorzugtes Metallchelat-gekoppeltes Lysinderivat gezeigt. In Abb. 2 ist ein biotinyliertes Lysinderivat gezeigt.

Der Begriff "Aktivester" im Sinne der vorliegenden Erfindung umfaßt aktivierte Estergruppen, die mit freien Aminogruppen von Peptiden unter solchen Bedingungen reagieren können, daß keine störenden Nebenreaktionen mit anderen reaktiven Gruppen des Peptids auftreten können. Vorzugsweise wird als Aktivesterderivat ein N-Hydroxysuccinimidester verwendet. Neben den N-Hydroxysuccinimidestern können auch analoge p-Nitrophenyl-, Pentafluorphenyl-, Imidazolyl- oder N-Hydroxybenzotriazolylester verwendet werden.

Die Herstellung von Hapten-, Markierungsgruppen- und Festphasenbindungsgruppenderivaten, die sich für den Einbau in Oligonukleotidträger eignen, ist bei Theisen et al. (Tetrahedron Letters 33 (1992), 5033-5036) am Beispiel des Fluoreszenzfarbstoffs 5-Carboxyfluorescein beschrieben, der zu einem Phosphoramiditderivat umgesetzt wird. Dieses Phosphoramiditderivat kann an das 5'-Ende oder/und das 3'-Ende von Oligonukleotiden oder innerhalb der Oligonukleotidsequenz eingebaut werden.

Vorzugsweise erfolgt die Einführung von Haptenen und Markierungs-bzw. Festphasenbindungsgruppen gemäß Variante (a), d.h. durch Verwendung von mit der jeweils einzuführenden Gruppe gekoppelten Monomerderivaten während der Festphasensynthese. Gemäß dieser Variante können beispielsweise lumineszierende Metallchelate, Biotin oder Peptidhaptene ohne weiteres eingeführt werden. Bei empfindlichen Fluoreszenzfarbstoffen oder -markierungen oder anderen Haptenen, wie etwa Steroiden, ist diese Vorgehensweise jedoch weniger bevorzugt, da diese Substanzen durch die bei der Festphasensynthese herrschenden Bedingungen zerstört werden können. In diesem Falle erfolgt die Herstellung der Konjugate vorzugsweise gemäß Verfahrensvariante (b), d.h. durch nachträgliche Kopplung an das fertige Trägermolekül. Natürlich ist eine Kombination der Verfahrensvarianten (a) und (b) möglich.

Auch die Einführung zwei verschiedener Gruppen gemäß Variante (b), d.h. nach Beendigung der Synthese, z.B. eines Haptens und einer Markierungsgruppe oder eines Hapten und einer Festphasenbindungsgruppe, ist möglich. Hierzu kann das Verfahren nach Variante (b) beispielsweise derart durchgeführt werden, daß die erste einzuführende Gruppe an Amino- und die zweite einzuführende Gruppe an Thiolseitengruppen des Trägermoleküls gekoppelt wird. Andererseits können beide einzuführenden Gruppen jeweils selektiv an vorbestimmte primäre Aminoseitengruppen des Trägers gekoppelt werden, wobei an Positionen des Trägers, an denen eine Kopplung mit Haptenmolekülen erfolgen soll, ein Monomerderivat mit einer ersten Schutzgruppe für die Aminoseitengruppe verwendet wird, und an Positionen des Trägers, an denen eine Kopplung mit Markierungs- bzw. Festphasenbindungsgruppen erfolgen soll, ein Monomerderivat mit einer zweiten Schutzgruppe für die Aminoseitengruppe verwendet wird und die erste und die zweite Schutzgruppe so ausgewählt werden, daß eine selektive Schutzgruppenabspaltung und demzufolge eine selektive Kopplung in zwei Reaktionsschritten möglich ist. Hierzu können die erste und zweite Schutzgruppe aus säurelabilen Aminoschutzgruppen, wie etwa Boc bzw. säurestabilen Schutzgruppen, wie etwa Phenylacetyl ausgewählt werden.

Bei dem erfindungsgemäßen Verfahren wird das Trägermolekül mit der gewünschten Monomersequenz an einer Festphase hergestellt. Die Herstellung von Peptidträgern erfolgt vorzugsweise mit einem kommerziellen Peptid-Synthesegerät (z.B. die Geräte A 431 oder A 433 von Applied Biosystems). Die Synthese erfolgt nach bekannten Methoden, vorzugsweise ausgehend vom Carboxylterminus des Peptids unter Verwendung von Aminosäurederivaten. Vorzugsweise werden Aminosäurederivate eingesetzt, deren für die Kupplung benötigte Amino-Endgruppe mit einem Fluorenylmethyloxycarbonyl (Fmoc)-Rest derivatisiert ist. Reaktive Seitengruppen der eingesetzten Aminosäuren enthalten Schutzgruppen, die nach Beendigung der Peptidsynthese ohne weiteres abspaltbar sind. Bevorzugte Beispiele hierfür sind Schutzgruppen, wie etwa Triphenylmethyl (Trt), t-Butylether (tBu), t-Butylester (OtBu), tert.-Butoxycarbonyl (Boc), 2,2,5,7,8-Pentamethylchroman-6-sulfonyl (Pmc) oder Phenylacetyl.

Die Aminoseitenketten von Lysinresten oder anderen Aminosäurederivaten mit primären Aminoseitengruppen, die sich an Positionen des Peptids befinden, an denen ein Hapten oder eine Markierung eingeführt werden soll, sind gemäß Variante (a) kovalent mit der einzuführenden Gruppe gekoppelt.

Neben den 20 natürlichen Aminosäuren kann das Peptid auch artifizielle Aminosäuren, wie etwa β-Alanin, γ-Aminobuttersäure, ε-Aminocapronsäure, Norleucin oder Ornithin enthalten. Diese artifiziellen Aminosäuren werden analog wie die natürlichen Aminosäuren für die Synthese in geschützter Form eingesetzt.

Gemäß Variante (b) des erfindungsgemäßen Verfahrens erfolgt nach Beendigung der Synthese die Einführung des Haptens bzw. der Markierung durch Umsetzung des Peptids nach Schutzgruppenabspaltung mit der jeweils gewünschten aktivierten Gruppe, die mit freien primären Aminogruppen des Peptids reagiert. Pro freie primäre Aminogruppe werden vorzugsweise 1,5 bis 4 Äquivalente Aktivester eingesetzt. Anschließend wird das Reaktionsprodukt aufgereinigt, vorzugsweise durch HPLC. Die Einführung von zwei unterschiedlichen aktivierten Gruppen gemäß Variante (b) gelingt durch Verwendung von zwei selektiv abspaltbaren Schutzgruppen, wie oben erläutert.

Das Peptidrückgrat des Konjugats besitzt eine nicht immunologisch reaktive Aminosäuresequenz, d.h. eine Aminosäuresequenz, die bei der vorgesehenen Anwendung des Konjugats als Antigen in einem immunologischen Nachweisverfahren die Durchführung des Tests nicht beeinträchtigt.

Andererseits kann das Rückgrat des Trägermoleküls auch aus Nukleotiden oder peptidischen Nukleinsäuren aufgebaut sein. Die Synthese eines Oligonukleotidträgermoleküls kann in einem kommerziellen DNA-Synthesegerät erfolgen. Die Haptenmoleküle und die Markierungs- bzw. Festphasenbindungsgruppen werden vorzugsweise als Phosphoramiditderivate eingeführt (Theisen et al., supra; Applied Biosystems, User Bulletin 67, FAM Amidite, Mai 1992) oder/und nachträglich an freie reaktive Seitengruppen gekoppelt. Die Synthese von Trägermolekülen auf Basis peptidischer Nukleinsäuren erfolgt analog einer Festphasenpeptidsynthese, z.B. gemäß der in WO92/20703 beschriebenen Methode. Die Einführung von Haptenmolekülen oder Markierungs- bzw. Festphasenbindungsgruppen kann entsprechend den für Peptid- und Oligonukleotidträger beschriebenen Methoden erfolgen.

Ein Gegenstand der vorliegenden Erfindung ist auch die Verwendung der Konjugate als Antigene in einem immunologischen Verfahren, wenn das Haptenmolekül ein immunologisch reaktives Molekül ist, oder zur DNA-Diagnostik, wenn das Hapten eine Nukleinsäure ist.

Konjugate, die mehr als 1 Haptenmolekül enthalten, können in immunologischen Nachweisverfahren als Polyhaptene eingesetzt werden.

Eine bevorzugte Ausführungsform der Erfindung betrifft die Verwendung der Konjugate in einem immunologischen Verfahren zur Bestimmung von spezifischen Antikörpern in einer Probeflüssigkeit. Vorzugsweise werden solche Antikörper bestimmt, die auf eine Infektion durch Mikroorganismen, wie etwa Bakterien, Viren oder Protozoen, hinweisen. Besonders bevorzugt werden gegen Viren gerichtete Antikörper, z.B. gegen HIV oder Hepatitis-Viren gerichtete Antikörper bestimmt. Die Probeflüssigkeit ist vorzugsweise Serum, besonders bevorzugt humanes Serum. Weiterhin ist bevorzugt, daß die erfindungsgemäßen Konjugate bei einem immunologischen Verfahren im Brückentestformat eingesetzt werden.

Ein Gegenstand der vorliegenden Erfindung ist somit ein Verfahren zur immunologischen Bestimmung eines spezifischen Antikörpers in einer Probeflüssigkeit, welches dadurch gekennzeichnet ist, daß man (a) die Probeflüssigkeit mit mindestens einem erfindungsgemäßen Konjugat, das gegen den zu bestimmenden. Antikörper gerichtet ist, inkubiert und (b) den Antikörper über eine Bindung Konjugat nachweist.

Das erfindungsgemäße immunologische Bestimmungsverfahren kann an sich nach jedem bekannten Testformat erfolgen, z.B. in einem homogenen Immunoassay mit einer einzigen Reaktionsphase oder in einem heterogenen Immunoassay mit mehr als einer Reaktionsphase. Vorzugsweise wird ein heterogenes Testformat verwendet, bei dem das Vorhandensein des Antikörpers in Anwesenheit einer Festphase nachgewiesen wird. Eine Ausführungsform dieses Testformats ist das sogenannte Doppelantigen-Brücken-Testkonzept. Hierbei wird die Probeflüssigkeit in Gegenwart einer reaktiven Festphase mit zwei gegen den zu bestimmenden Antikörper gerichteten Antigenen inkubiert, wobei das erste Antigen eine Markierungsgruppe trägt und das zweite Antigen an die Festphase gebunden ist oder in einer an die Festphase bindefähigen Form vorliegt. Das erste oder/und das zweite Antigen ist ein erfindungsgemäßes Konjugat. Der zu bestimmende Antikörper in der Probeflüssigkeit wird gegebenenfalls nach Abtrennen der Festphase von der Inkubationsflüssigkeit durch Bestimmung der Markierung in der Festphase oder/und in der flüssigen Phase nachgewiesen. Vorzugsweise ist das erste Antigen ein mit einem lumineszierenden Metallchelat oder einer Fluoreszenzgruppe markiertes Konjugat. Das zweite Antigen ist vorzugsweise mit Biotin markiert und ist an eine Festphase bindefähig, die mit Streptavidin oder Avidin beschichtet ist.

Die Testdurchführung beinhaltet vorzugsweise ein Mischen der Probeflüssigkeit mit dem ersten markierten Antigen und dem festphasenseitigen zweiten Antigen, um einen markierten, immobilisierten Komplex aus erstem Antigen, Antikörper und festphasengebundenem zweiten Antigen zu erhalten. Gegenüber anderen Testformaten zum Nachweis von Antikörpern führt das Brückentestformat sowohl zu einer Verbesserung der Sensitivität, d.h. es werden alle Immunglobulinklassen, wie etwa IgG, IgM, IgA und IgE, erkannt, als auch der Spezifität, d.h. es wird die unspezifische Reaktivität verringert.

Eine zweite bevorzugte Ausführungsform der Erfindung betrifft die Verwendung der Konjugate in einem kompetitiven Immunoassay. Kompetitive Immunoassays werden im allgemeinen zum Nachweis von niedermolekularen Analyten verwendet und können prinzipiell in zwei Testformaten, dem "labelled antibody" Format und dem "labelled analog" Format durchgeführt werden. Beim "labelled antibody" Format wird die den nachzuweisenden Analyten enthaltende Probeflüssigkeit mit einem an eine Festphase bindefähigen Hapten, das mit dem Analyten immunologisch konkurriert, und einen markierten, gegen das Hapten und den Analyten gerichteten Rezeptor, z.B. einen Antikörper, markiert. Bei diesem Testformat ist die an die Festphase gebundene Markierung umgekehrt proportional zur Konzentration des Analyten. Beim "labelled analog" Format wird die den nachzuweisenden Analyten enthaltende Probeflüssigkeit mit einem markierten, mit dem Analyten immunologisch konkurrierenden Hapten und einem gegen den Analyten und das Hapten gerichteten Rezeptor, der an eine Festphase bindefähig ist, inkubiert. Die Menge an festphasengebundenem markiertem Hapten ist dann der Konzentration an freiem, zu bestimmendem Analyten umgekehrt proportional.

Ein Gegenstand der vorliegenden Erfindung ist somit ein Verfahren zum Nachweis eines Analyten in einer Probeflüssigkeit nach dem Prinzip des kompetitiven Immunoassay im "labelled analog" Format, dadurch gekennzeichnet, daß man (a) die Probeflüssigkeit in Gegenwart einer reaktiven Festphase mit einem erfindungsgemäßen Konjugat, das eine Markierungsgruppe enthält, und einem Rezeptor, der an die Festphase gebunden oder zur Bindung an eine Festphase fähig ist und eine spezifisch immunologische Reaktion mit dem Analyten und der Hapten- komponente des Konjugats eingehen kann, inkubiert, (b) die Festphase gegebenenfalls von der Inkubationsflüssigkeit abtrennt und (c) das Vorhandensein oder/und die Menge des Analyten in der Probeflüssigkeit durch Messung der Markierungskomponente des Konjugats in der Festphase oder/und in der Inkubationsflüssigkeit bestimmt. Als immobilisierbaren Rezeptor verwendet man vorzugsweise einen biotinylierten Antikörper bzw. ein biotinyliertes Antikörperfragment und als reaktive Festphase eine mit Streptavidin oder Avidin beschichtete Festphase.

Noch ein weiterer Gegenstand der vorliegenden Erfindung ist ein Verfahren zum Nachweis eines Analyten in einer Probeflüssigkeit nach dem Prinzip des kompetitiven Immunoassay im "labelled antibody" Format, dadurch gekennzeichnet, daß man (a) die Probeflüssigkeit in Gegenwart einer reaktiven Festphase mit einem erfindungsgemäßen Konjugat, das eine Festphasenbindungsgruppe enthält, und einem Rezeptor, der eine Markierungsgruppe trägt und eine spezifisch immunologische Reaktion mit dem Analyten und der Haptenkomponente des Konjugats eingehen kann, inkubiert, (b) die Festphase gegebenenfalls von der Inkubationsflüssigkeit abtrennt und (c) das Vorhandensein oder/und die Menge des Analyten in der Probeflüssigkeit durch Messung der Markierungskomponente des Rezeptors an der Festphase oder/und in der Inkubationsflüssigkeit bestimmt. Vorzugsweise verwendet man ein biotinyliertes Konjugat und eine mit Streptavidin oder Avidin beschichtete Festphase. Als markierten Rezeptor verwendet man vorzugsweise einen Antikörper bzw. ein Antikörperfragment.

Der Nachweis von lumineszierenden Metallchelatgruppen erfolgt vorzugsweise durch Elektrochemilumineszenz, wobei lumineszierende Spezies elektrochemisch an der Oberfläche einer Elektrode erzeugt werden. Der Nachweis der Lumineszenz kann qualitativ oder/und quantitativ erfolgen. Beispiele zur Durchführung von Lumineszenz-Assays finden sich in EP-A-0 580 979, WO 90/05301, WO 90/11511 und WO 92/14138. Auf die dort offenbarten Verfahren und Vorrichtungen für Lumineszenz-Assays wird hiermit Bezug genommen. Die Festphase in Elektrochemilumineszenz-Assays besteht vorzugsweise aus Mikropartikeln, besonders bevorzugt magnetischen Mikropartikeln, die mit einer Beschichtung versehen sind, die mit dem festphasenseitigen zweiten Antigen wechselwirkt. Vorzugsweise sind die Mikropartikel mit Streptavidin beschichtet.

Die Elektrochemilumineszenz-Messung wird vorzugsweise in Gegenwart eines Reduktionsmittels für den Metallkomplex durchgeführt, z.B. einem Amin. Bevorzugt sind aliphatische Amine, insbesondere primäre, sekundäre und tertiäre Alkylamine, deren Alkylgruppen jeweils ein bis drei Kohlenstoffatome aufweist. Besonders bevorzugt ist Tripropylamin. Das Amin kann jedoch auch ein aromatisches Amin, wie Anilin oder ein heterocyklisches Amin sein.

Weiterhin kann gegebenenfalls als Verstärker ein nichtionisches oberflächenaktives Mittel, z.B. ein ethoxyliertes Phenol vorhanden sein. Derartige Substanzen sind beispielsweise kommerziell unter den Bezeichnungen Triton X100 oder Triton N-401 erhältlich.

Andererseits kann der Nachweis der lumineszierenden Metallchelatgruppe auch durch Fluoreszenz erfolgen, wobei das Metallchelat durch Bestrahlung mit einem Licht der geeigneten Wellenlänge angeregt und die daraus resultierende Fluoreszenzstrahlung gemessen wird. Beispiele zur Durchführung von Fluoreszenz-Assays finden sich in EP-A-0 178 450 und EP-A-0 255 534. Auf diese Offenbarung wird hiermit Bezug genommen.

Der Nachweis von Fluoreszenzgruppen, die ebenso wie die lumineszierenden Metallchelate zu den bevorzugten Markierungsgruppen der erfindungsgemäßen Konjugate gehören, kann - wie zuvor ausgeführt - durch Anregung und Messung der Fluoreszenz auf bekannte Weise erfolgen.

Noch ein weiterer Gegenstand der vorliegenden Erfindung ist ein immunologisches Reagenz, das mindestens ein erfindungsgemäßes markiertes oder festphasenbindefähiges Konjugat enthält. Ein Reagenz zur immunologischen Bestimmung eines spezifischen Antikörpers nach dem Prinzip des Doppelantigen-Brückentests enthält (a) ein markiertes erfindungsgemäßes Konjugat oder/und (b) ein weiteres erfindungsgemäßes Konjugat, das an eine Festphase gebunden ist oder in einer an eine Festphase bindefähigen Form vorliegt.

Ein Reagenz zur Bestimmung eines Analyten, vorzugsweise eines niedermolekularen Analyten nach dem Prinzip des kompetitiven Immunoassays enthält ein markiertes Konjugat ("labelled analog"-Format) oder ein festphasenbindefähiges Konjugat ("labelled antibody"-Format), das mit dem zu bestimmenden Analyten immunologisch um die Bindung an einen Rezeptor konkurriert. Vorzugsweise enthält das Reagenz für einen kompetitiven Immunoassay räumlich getrennt vom erfindungsgemäßen Konjugat entweder einen markierten Rezeptor ("labelled antibody"-Format) oder einen festphasenbindefähigen Rezeptor ("labelled analog"-Format), der mit dem zu bestimmenden Analyten und dem erfindungsgemäßen Konjugat eine immunologische Reaktion eingehen kann.

Die Erfindung wird weiterhin durch die folgenden Beispiele und Abbildungen erläutert. Es zeigen:
- Abb. 1: ein Metallchelat-Lysin-Derivat,
- Abb. 2: ein Biotin-Lysin-Derivat,
- Abb. 3: ein erfindungsgemäßes Konjugat
- Abb. 4: ein Vergleichskonjugat.

### Beispiel 1

### Herstellung eines Metallchelat-Lysin-Derivats

6 mmol des Rutheniumkomplexes Ru(Bipyridin)₂ (Bipyridin-CO-N-hydroxysuccinimidester) gemäß EP-A-0 580 979 wurden in 50 ml Dimethylformamid gelöst und dazu eine Lösung von α-Fmoc-Lysin getropft. Nach Abziehen des Lösungsmittels wurde der Rückstand in wenig Aceton gelöst, mit 300 ml Chloroform versetzt und kurz zum Sieden erhitzt. Nach Abtrennen des Lösungsmittels wurde die in Abb. 1 gezeigte Verbindung als Feststoff erhalten.

### Beispiel 2

Herstellung von Metallchelat-markierten und biotinylierten Peptiden

Die Metallchelat-markierten bzw. biotinylierten Peptide wurden mittels Fluorenylmethyloxycarbonyl-(Fmoc)-Festphasenpeptidsynthese an einem Batch-Peptidsynthesizer, z.B. von Applied Biosystems A431 oder A433, hergestellt.. Dazu wurden jeweils 4.0 Äquivalente der in Tabelle 1 dargestellten Aminosäurederivate verwendet:

**Tabelle 1:**

| | |
|---|---|
| A | Fmoc-Ala-OH |
| C | Fmoc-Cys(Trt)-OH |
| D | Fmoc-Asp(OtBu)-OH |
| E | Fmoc-Glu(OtBu)-OH |
| F | Fmoc-Phe-OH |
| G | Fmoc-Gly-OH |
| H | Fmoc-His(Trt)-OH |
| I | Fmoc-Ile-OH |
| K | Fmoc-Lys(Boc)-OH |
| L | Fmoc-Leu-OH |
| M | Fmoc-Met-OH |
| N | Fmoc-Asn(Trt)-OH |
| P | Fmoc-Pro-OH |
| Q | Fmoc-Gln(Trt)-OH |
| R | Fmoc-Arg(Pmc)-OH |
| S | Fmoc-Ser(tBu)-OH |
| T | Fmoc-Thr(tBu)-OH |
| U | Fmoc-βAlanin-OH |
| V | Fmoc-Val-OH |
| W | Fmoc-Trp-OH |
| Y | Fmoc-Tyr(tBu)-OH |
| Z | Fmoc-ε-Aminocapronsäure-OH |
| Nle | Fmoc-ε-Norleucin-OH |
| Abu | Fmoc-γ-Aminobuttersäure-OH |

Die Einführung von Metallchelat- und Biotingruppen in die Peptidsequenz erfolgte durch direkten Einbau von Metallchelatoder Biotin-gekoppelten Aminosäurederivaten, z.B. innerhalb der Sequenz über einen mit Metallchelat-Aktivester ∈-derivatisierten Lysinrest (Abb. 1) bzw. über einen mit Biotin derivatisierten Lysinrest (Abb. 2) oder N-terminal durch Verwendung eines entsprechenden α-derivatisierten Aminosäurerests.

Die Aminosäuren oder Aminosäurederivate wurden in N-Methylpyrrolidon gelöst. Das Peptid wurde an 400-500 mg 4-(2',4'-Dimethoxyphenyl-Fmoc-Aminomethyl)-Phenoxy-Harz (Tetrahedron Letters 28 (1987), 2107) mit einer Beladung von 0,4-0,7 mmol/g aufgebaut (JACS 95 (1973), 1328). Die Kupplungsreaktionen wurden bezüglich des Fmoc-Aminosäurederivats mit 4 Äquivalenten Dicyclohexylcarbodiimid und 4 Äquivalenten N-Hydroxybenzotriazol in Dimethylformamid als Reaktionsmedium während 20 min durchgeführt. Nach jedem Syntheseschritt wurde die Fmoc-Gruppe mit 20%igem Piperidin in Dimethylformamid in 20 min abgespalten.

Die Freisetzung des Peptids vom Träger und die Abspaltung der säurelabilen Schutzgruppen erfolgte mit 20 ml Trifluoressigsäure, 0,5 ml Ethandithiol, 1 ml Thioanisol, 1,5 g Phenol und 1 ml Wasser in 40 min bei Raumtemperatur. Die Reaktionslösung wurde anschließend mit 300 ml gekühltem Diisopropylether versetzt und zur vollständigen Fällung des Peptids 40 min bei 0°C gehalten. Der Niederschlag wurde abfiltriert, mit Diisopropylether nachgewaschen, mit wenig 50 %-iger Essigsäure gelöst und lyophilisiert.

Das erhaltene Rohmaterial wurde mittels präparativer HPLC an Delta-PAK RP C18-Material (Säule 50 x 300 mm, 100 Å, 15 µ) über einen entsprechenden Gradienten (Eluent A: Wasser, 0,1% Trifluoressigsäure, Eluent B: Acetonitril, 0,1% Trifluoressigsäure) in ca. 120 min. aufgereinigt. Die Identität des eluierten Materials wurde mittels Ionenspray-Massenspektrometrie geprüft.

Die Entfernung von säurestabilen Phenylacetylschutzgruppen erfolgte enzymatisch mit immobilisierter oder löslicher Penicillin G-Amidase in wäßriger Lösung mit organischem Solvensanteil bei Raumtemperatur.

### Beispiel 3

### Kopplung mit Haptenen

Das Metallchelat-markierte bzw. biotinylierte Peptid wurde in Dimethylformamid gelöst und bezüglich der kupplungsfähigen Positionen des Peptids (z.B. Aminoseitengruppen von Lysin) jeweils ein leichter Überschuß (ca. 30 %) des aktivierten Haptens (z.B. N-Hydroxysuccinimidester) zugefügt. Es wurde für 1 Stunde bei Raumtemperatur gerührt, das Lösungsmittel im Hochvakuum abgezogen und das Peptid über präparative HPLC gereinigt.

Unter Verwendung des in Abb. 1 gezeigten Metallchelat-Lysin-Derivats als Markierungsgruppe und von Estradiol (E2) als Hapten wurden Konjugate mit der folgenden Struktur hergestellt:
I:AcK(BPRu)UEUEUK(E2)UEUEUK(BPRu)UEUK(E2)-NH₂
II:AcK(BPRu)UEUEUK(E2)UEUEUK(BPRu)U-NH₂

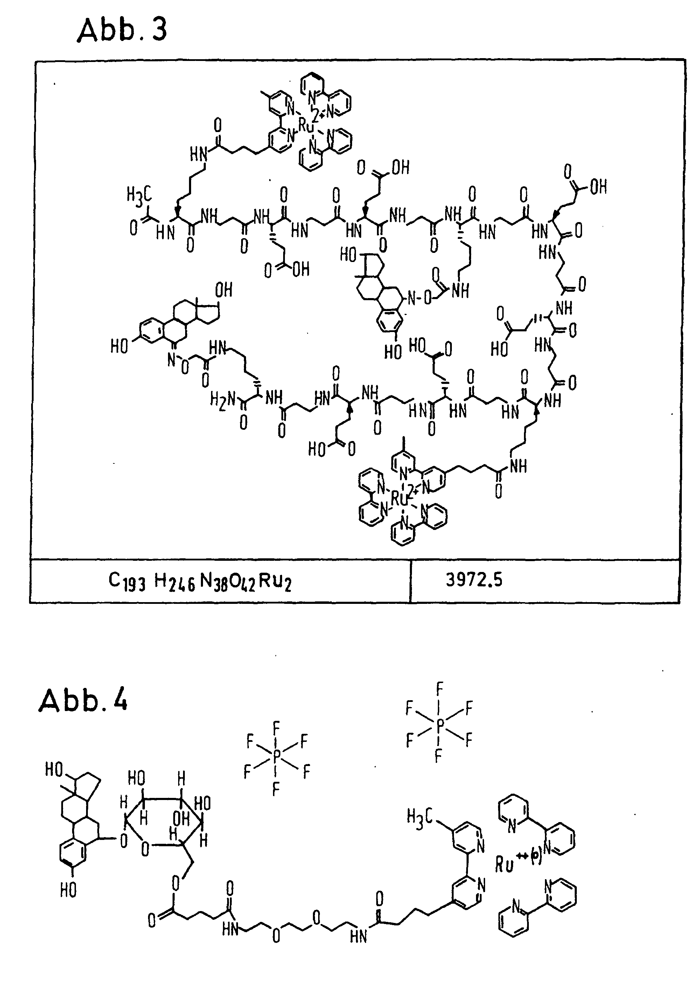

Das Konjugat I ist in Abb. 3 dargestellt. Der Aminoterminus der Peptidketten ist durch Acetyl (Ac) geschützt. Der Carboxylterminus liegt als Säureamidgruppe vor.

Die Metallchelat- und Haptenmoleküle sind jeweils über die ∈-Aminoseitengruppe der Lysine an die Peptidkette gekoppelt.

Auf analoge Weise wurde unter Verwendung von Testosteron als Haptenmolekül ein Konjugat mit der Sequenz von Konjugat I synthetisiert.

Die Struktur der hergestellten Konjugate wurde durch ¹H-NMR (500 MHz) überprüft und bestätigt.

### Beispiel 4

### Bestimmung von Estradiol in Serum

Es wurde ein kompetitiver Zweischritt-Assay nach dem "labelled analog"-Format zur Bestimmung von Estradiol in Humanserum durchgeführt. Hierzu wurden 90 µl Lösung 1 (0,69 nmol/l erfindungsgemäßes Konjugat I (Abb. 3) bzw. 1,68 nmol/l Vergleichskonjugat (Abb. 4), jeweils in 50 mmol/l 4-Morpholinethansulfonsäure (MES) , pH 6,8, 0,1 % Rinderserumalbumin, 0,1 % Thesit, 0,01 % Methylisothiazolon, 0,1 % Oxypyrion, 30 ng/ml Ablösereagenz Dihydrotestosteron) zusammen mit 50 µl Probe (Serumprobe oder Estradiol Standard) in einem Polystyrolgefäß 10 min bei 37°C inkubiert. Anschließend erfolgte die aufeinanderfolgende Zugabe von 90 µl Lösung 2 (0,7 µg/ml bzw. 1,4 µg/ml Konjugat aus Biotin und polyklonalem Anti-Estradiol-Kaninchen-Fab' in 50 mmol/l MES-Puffer pH 6,0) und 50 µl Bead-Suspension (720 µg/ml Streptavidinbeschichtete Magnetpartikel, Fa. Dynal).

Nach weiteren 10 min Inkubation bei 37°C wurden 150 µl der Mischung in eine Meßzelle überführt. Dort erfolgte eine magnetische Konzentrierung der Bead-Partikel und der daran haftenden Rutheniummarkierung an die Elektrodenoberfläche und eine Detektion des elektrochemisch erzeugten Chemilumineszenzsignals bei 28°C.

Das in Tabelle 2 dargestellte Ergebnis dieses Versuchs zeigt, daß das erfindungsgemäße Konjugat gegenüber dem Vergleichskonjugat eine deutliche Verbesserung der Testperformance hinsichtlich Sensitivität und unterer Nachweisgrenze bringt.

**Tabelle 2**

| Antigen | Konjugat I (Erfindung) | Konjugat (vergleich) |
|---|---|---|
| Antigenkonzentration (nmol/l) | 0,69 | 1,68 |
| Rutheniumkomplex-Konzentration (nmol/l) | 1,68 | 1,68 |
| Konzentration Antiserum (µg/ml) | 0,7 | 1,4 |
| Counts Standard F | 80412 | 87709 |
| Counts Standard B | 653420 | 1224219 |
| Counts Standard A | 861140 | 1445270 |
| Verhältnis B/A | 0,759 | 0,847 |
| Verhältnis F/A | 0,093 | 0,061 |
| Verhältnis F/E | 0,546 | 0,442 |
| untere Nachweisgrenze pg/ml (3 % VK) | 15,9 | 25,1 |

## Patentansprüche

1. Konjugat, umfassend einen polymeren Träger mit maximal 100 monomeren Einheiten, der gekoppelt an reaktive Gruppen 1-10 Haptenmoleküle und 1-10 Markierungs- oder Festphasenbindungsgruppen enthält, wobei die monomeren Einheiten aus Aminosäuren ausgewählt sind, und wobei die Haptenmoleküle und die Markierungs- oder Festphasenbindungsgruppen über die primären Amino- oder/und Thiolgruppe der Aminosäuremonomere an den polymeren Träger gekoppelt sind.

2. Konjugat nach Anspruch 1,
**dadurch gekennzeichnet,**
**dass** der polymere Träger eine Länge von 3-80 monomere Einheiten aufweist.

3. Konjugat nach einem der Ansprüche 1 bis 2,
**dadurch gekennzeichnet,**
**dass** es Markierungsgruppen enthält, die aus lumineszierenden Metallchelaten oder Fluoreszenzgruppen ausgewählt sind.

4. Konjugat nach einem der Ansprüche 1 bis 3,
**dadurch gekennzeichnet,**
**dass** es Festphasenbindungsgruppen enthält, die aus Biotin und Biotinanaloga ausgewählt sind.

5. Konjugat nach einem der Ansprüche 1 bis 3,
**dadurch gekennzeichnet,**
**dass** der polymere Träger mindestens einen positiven oder/und negativen Ladungsträger enthält.

6. Konjugat nach Anspruch 5,
**dadurch gekennzeichnet,**
**dass** der polymere Träger als negativen Ladungsträger Carboxylatgruppen enthält.

7. Konjugat nach einem der vorhergehenden Ansprüche,
**dadurch gekennzeichnet,**
**dass** das Hapten ein immunologisch reaktives Molekül mit einer Molekularmasse von 100-2000 Da, ausgewählt aus pharmakologischen Wirkstoffen, Hormonen, Metaboliten, Vitaminen, Mediatoren und Neurotransmittern, ist.

8. Konjugat nach einem der vorhergehenden Ansprüche,
**dadurch gekennzeichnet,**
**dass** die monomeren Einheiten des Träger artifizielle Aminosäuren enthalten.

9. Konjugat nach Anspruch 8,
**dadurch gekennzeichnet,**
**dass** die artifiziellen Aminosäuren ausgewählt sind aus β-Alanin, γ-Aminobuttersäure, ε-Aminocaprosäure, Norleucin und Ornithin.

10. Verfahren zur Herstellung von Konjugaten, umfassend einen polymeren Träger mit maximal 100 monomeren Einheiten, der gekoppelt an reaktive Gruppen 1-10 Haptenmoleküle und 1-10 Markierungs- oder Festphasenbindungsgruppen enthält, wobei die monomeren Einheiten aus Aminosäuren, Nukleotiden, Nukleotidanaloga und peptidischen Nukleinsäuren ausgewählt sind,
**dadurch gekennzeichnet,**
**dass** man einen polymeren Träger aus monomeren Einheiten an einer Festphase synthetisiert, wobei man
(a) während der Synthese an vorbestimmten Positionen des Trägers Monomerderivate einführt, die kovalent mit Haptenmolekülen oder/und Markierungs- bzw. Festphasenbindungsgruppen gekoppelt sind, oder/und
(b) nach der Synthese aktivierte Haptenmoleküle oder/und Markierungs-bzw. Festphasenbindungsgruppen an reaktive Gruppen des Trägers koppelt,
wobei in Variante (b) die Kopplung an die primären Amino- oder/und Thiol-Gruppen der Aminosäuremonomere nach Abspaltung von Schutzgruppen der zur Festphasensynthese verwendeten Monomerderivate erfolgt.

11. Verfahren nach Anspruch 10,
**dadurch gekennzeichnet,**
**dass** man einen Peptidträger aus Aminosäurederivaten synthetisiert.

12. Verfahren nach Anspruch 10 oder 11,
**dadurch gekennzeichnet,**
**dass** in Variante (a) die Haptenmoleküle oder/und Markierungs- bzw. Festphasenbindungsgruppen jeweils an eine primäre Aminogruppe oder eine Thiolgruppe des Monomerderivats gekoppelt sind.

13. Verfahren nach einem der Ansprüche 10 bis 12,
**dadurch gekennzeichnet,**
**dass** man nach der Synthese in Variante (b) die Haptenmoleküle und Markierungs- bzw. Festphasenbindungsgruppen an primäre Aminoseitengruppen des Trägers koppelt, wobei an Positionen des Trägers, an denen eine Kopplung mit Haptenmolekülen erfolgen soll, ein Monomerderivat mit einer ersten Schutzgruppe für die Amino-Seitengruppe verwendet wird, und an Positionen des Trägers, an denen eine Kopplung mit Markierungs- bzw. Festphasenbindungsgruppen erfolgen soll, ein Monomerderivat mit einer zweiten Schutzgruppe für die Aminoseitengruppe verwendet wird und die erste und die zweite Schutzgruppe so ausgewählt werden, dass eine selektive Schutzgruppenabspaltung möglich ist.

14. Verfahren nach Anspruch 13,
**dadurch gekennzeichnet,**
**dass** die erste und zweite Schutzgruppe aus säurelabilen bzw. säurestabilen Schutzgruppen ausgewählt werden.

15. Verwendung von Konjugaten nach einem der Ansprüche 1 bis 9 als Antigen in einem immunologischen Verfahren oder zur Nukleinsäure-Diagnostik.

16. Verwendung nach Anspruch 15,
**dadurch gekennzeichnet,**
**dass** man Konjugate, die mehr als 1 Haptenmolekül enthalten, als Polyhaptene in immunologischen Nachweisverfahren einsetzt.

17. Verwendung nach Anspruch 15 oder 16 in einem kompetitiven Immunoassay.

18. Verwendung nach Anspruch 15 oder 16 in einem Immunoassay zum Nachweis spezifischer Antikörper.

19. Verfahren zum Nachweis eines Analyten in einer Probeflüssigkeit nach dem Prinzip des kompetitiven Immunoassay im "labelled analog" Format,
**dadurch gekennzeichnet,**
**dass** man
(a) die Probeflüssigkeit in Gegenwart einer reaktiven Festphase mit einem Konjugat nach einem der Ansprüche 1 bis 9, das eine Markierungsgruppe enthält, und einem Rezeptor, der an die Festphase gebunden oder zur Bindung an eine Festphase fähig ist und eine spezifisch immunologische Reaktion mit dem Analyten und der Haptenkomponente des Konjugats eingehen kann, inkubiert,
(b) die Festphase gegebenenfalls von der Inkubationsflüssigkeit abtrennt und
(c) das Vorhandensein oder/und die Menge des Analyten in der Probeflüssigkeit durch Messung der Markierungskomponente des Konjugats in der Festphase oder/und in der Inkubationsflüssigkeit bestimmt.

20. Verfahren nach Anspruch 19,
**dadurch gekennzeichnet,**
**dass** man als Rezeptor einen biotinylierten Antikörper bzw. ein biotinyliertes Antikörperfragment und eine mit Streptavidin oder Avidin beschichtete Festphase verwendet.

21. Verfahren zum Nachweis eines Analyten in einer Probeflüssigkeit nach dem Prinzip des kompetitiven Immunoassay im "labelled antibody" Format,
**dadurch gekennzeichnet,**
**dass** man
(a) die Probeflüssigkeit in Gegenwart einer reaktiven Festphase mit einem Konjugat nach einem der Ansprüche 1 bis 9 das eine Festphasenbindungsgruppe enthält, und einem Rezeptor, der eine Markierungsgruppe trägt und eine spezifisch immunologische Reaktion mit dem Analyten und der Haptenkomponente des Konjugats eingehen kann, inkubiert,
(b) die Festphase gegebenenfalls von der Inkubationsflüssigkeit abtrennt und ,
(c) das Vorhandensein oder/und die Menge des Analyten in der Probeflüssigkeit durch Messung der Markierungskomponente des Rezeptors in der Festphase oder/und in der Inkubationsflüssigkeit bestimmt.

22. Verfahren nach Anspruch 21,
**dadurch gekennzeichnet,**
**dass** man ein biotinyliertes Konjugat und eine mit Streptavidin oder Avidin beschichtete Festphase verwendet.

23. Verfahren zum Nachweis eines spezifischen Antikörpers in einer Probeflüssigkeit,
**dadurch gekennzeichnet,**
**dass** man
(a) die Probeflüssigkeit mit mindestens einem Konjugat nach einem der Ansprüche 1 bis 9, das gegen den zu bestimmenden Antikörper gerichtet ist, inkubiert und
(b) den Antikörper über eine Bindung mit dem Konjugat nachweist.

24. Verfahren nach Anspruch 23,
**dadurch gekennzeichnet,**
**dass** man
(a) die Probeflüssigkeit in Gegenwart einer reaktiven Festphase und zwei gegen den zu bestimmenden Antikörper gerichteten Antigenen inkubiert, wobei das erste Antigen eine Markierungsgruppe trägt und das zweite Antigen an die Festphase gebunden oder in einer an die Festphase bindefähigen Form vorliegt,
(b) die Festphase gegebenenfalls von der Inkubationsflüssigkeit abtrennt und
(c) das Vorhandensein oder/und die Menge des Antikörpers durch Bestimmung der Markierung in der Festphase oder/und in der flüssigen Phase nachweist,
wobei man als erstes oder/und zweites Antigen ein Konjugat nach einem der Ansprüche 1 bis 9 verwendet.

25. Verfahren nach Anspruch 24,
**dadurch gekennzeichnet,**
**dass** man als erstes Antigen ein mit einem lumineszierenden Metallchelat oder einer Fluoreszenzgruppe markiertes Konjugat verwendet.

26. Verfahren nach Anspruch 24 oder 25
**dadurch gekennzeichnet,**
**dass** man als zweites Antigen ein biotinyliertes Konjugat und eine mit Streptavidin oder Avidin beschichtete Festphase verwendet.

## Claims

1. Conjugate comprising a polymeric carrier with a maximum of 100 monomeric units which contains 1 - 10 hapten molecules and 1 - 10 marker or solid phase binding groups coupled to reactive groups, wherein the monomeric units are selected from amino acids, and the hapten molecules and the marker or solid phase binding groups are coupled to the polymeric carrier via the primary amino or/and thiol groups of the amino acid monomers.

2. Conjugate as claimed in claim 1,
**characterized in that**
the polymeric carrier has a length of 3 - 80 monomeric units.

3. Conjugate as claimed in one of the claims 1 to 2,
**characterized in that**
it contains marker groups which are selected from luminescent metal chelates or fluorescent groups.

4. Conjugate as claimed in one of the claims 1 to 3,
**characterized in that**
it contains solid phase binding groups that are selected from biotin or biotin analogues.

5. Conjugate as claimed in one of the claims 1 to 3,
**characterized in that**
the polymeric carrier contains at least one positive or/and negative charge carrier.

6. Conjugate as claimed in claim 5,
**characterized in that**
the polymeric carrier contains carboxylate groups as negative charge carriers.

7. Conjugate as claimed in one of the previous claims,
**characterized in that**
the hapten is an immunologically reactive molecule having a molecular mass of 100-2000 Da selected from pharmacologically active substances, hormones, metabolites, vitamins, mediators and neurotransmitters.

8. Conjugate as claimed in one of the previous claims,
**characterized in that**
the monomeric units of the carrier contain artificial amino acids.

9. Conjugate as claimed in claim 8,
**characterized in that**
the artificial amino acids are selected from β-alanine, γ-amino-butyric acid, ε-amino-caproic acid, norleucine or omithine.

10. Process for the production of conjugates comprising a polymeric carrier with a maximum of 100 monomeric units which contains 1 - 10 hapten molecules and 1 - 10 marker or solid phase binding groups coupled to reactive groups, the monomeric units being selected from amino acids, nucleotides, nucleotide analogues and peptidic nucleic acids
**characterized in that**
a polymeric carrier composed of monomeric units is synthesized on a solid phase in which
(a) during the synthesis monomer derivatives are introduced at predetermined positions on the carrier which are covalently coupled to hapten molecules or/and marker or solid phase binding groups or/and
(b) after the synthesis activated hapten molecules or/and marker or solid phase binding groups are coupled to reactive groups of the carrier,
and in variant (b) the coupling to the primary amino or/and thiol groups of the amino acid monomers takes place after cleaving protecting groups from the monomer derivatives used for the solid phase synthesis.

11. Process as claimed in claim 10,
**characterized in that**
a peptide carrier is synthesized from amino acid derivatives.

12. Process as claimed in claim 10 or 11,
**characterized in that**
in variant (a) the hapten molecules or/and marker groups or solid phase binding groups are in each case coupled to a primary amino group or a thiol group of the monomer derivative.

13. Process as claimed in one of the claims 10 to 12,
**characterized in that**
after the synthesis in variant (b) the hapten molecules and marker or solid phase binding groups are coupled to primary amino side groups of the carrier, wherein a monomer derivative with a first protecting group for the amino side group is used at positions of the carrier at which the hapten molecules are to be coupled and a monomer derivative with a second protecting group for the amino side group is used at positions of the carrier at which marker or solid phase binding groups are to be coupled and the first and the second protecting group are selected in such a way that it is possible to selectively cleave the protecting groups.

14. Process as claimed in claim 13,
**characterized in that**
the first and second protecting group are selected from acid-labile or acid-stable protecting groups.

15. Use of conjugates as claimed in one of the claims 1 to 9 as an antigen in an immunological method or for nucleic acid diagnostics.

16. Use as claimed in claim 15,
**characterized in that**
conjugates that contain more than 1 hapten molecule are used as polyhaptens in immunological detection methods.

17. Use as claimed in claim 15 or 16 in a competitive immunoassay.

18. Use as claimed in claim 15 or 16 in an immunoassay for detecting specific antibodies.

19. Method for the detection of an analyte in a sample liquid based on the principle of a competitive immunoassay in a "labelled analogue" format,
**characterized in that**
(a) the sample liquid is incubated in the presence of a reactive solid phase with a conjugate as claimed in one of the claims 1 to 9 which contains a marker group and with a receptor which is bound to the solid phase or is capable of binding to a solid phase and can enter into a specific immunological reaction with the analyte and the hapten component of the conjugate,
(b) the solid phase is optionally separated from the incubation liquid and
(c) the presence or/and the amount of analyte in the sample liquid is determined by measuring the marker component of the conjugate in the solid phase or/and in the incubation liquid.

20. Method as claimed in claim 19,
**characterized in that**
a biotinylated antibody or a biotinylated antibody fragment is used as a receptor and a solid phase coated with streptavidin or avidin is used.

21. Method for the detection of an analyte in a sample liquid based on the principle of a competitive immunoassay in a "labelled antibody" format,
**characterized in that**
(a) the sample liquid is incubated in the presence of a reactive solid phase with a conjugate as claimed in one of the claims 1 to 9 which contains a solid phase binding group and with a receptor which carries a marker group and can enter into a specific immunological reaction with the analyte and the hapten component of the conjugate,
(b) the solid phase is optionally separated from the incubation liquid and
(c) the presence or/and the amount of analyte in the sample liquid is determined by measuring the marker component of the receptor in the solid phase or/and in the incubation liquid.

22. Method as claimed in claim 21,
**characterized in that**
a biotinylated conjugate and a solid phase coated with streptavidin or avidin is used.

23. Method for the detection of a specific antibody in a sample liquid,
**characterized in that**
(a) the sample liquid is incubated with at least one conjugate as claimed in one of the claims 1 to 9 which is directed against the antibody to be determined and
(b) the antibody is detected via binding to the conjugate.

24. Method as claimed in claim 23,
**characterized in that**
(a) the sample liquid is incubated in the presence of a reactive solid phase and two antigens directed against the antibody to be determined wherein the first antigen carries a marker group and the second antigen is bound to the solid phase or is present in a form capable of binding to the solid phase,
(b) the solid phase is optionally separated from the incubation liquid and
(c) the presence or/and the amount of the antibody is detected by
determining the label in the solid phase or/and in the liquid phase, wherein a conjugate as claimed in one of the claims 1 to 9 is used as the first or/and the second antigen.

25. Method as claimed in claim 24,
**characterized in that**
a conjugate labelled with a luminescent metal chelate or with a fluorescent group is used as the first antigen.

26. Method as claimed in claim 24 or 25,
**characterized in that**
a biotinylated conjugate or a solid phase coated with streptavidin or avidin is used as the second antigen.

## Revendications

1. Conjugué comprenant un support polymère ayant au maximum 100 unités monomères, qui contient 1-10 molécules d'haptène et 1-10 groupes marqueurs ou de liaison aux phases solides couplés à des groupes réactifs, les unités monomères étant choisies parmi les acides aminés, et les molécules d'haptène et les groupes marqueurs ou de liaison aux phases solides étant couplés au support polymère par les groupes thiol et/ou amine primaire des monomères d'acide aminé.

2. Conjugué selon la revendication 1, **caractérisé en ce que** le support polymère présente une longueur de 3-80 unités monomères.

3. Conjugué selon l'une quelconque des revendications 1 à 2, **caractérisé en ce qu'**il contient des groupes marqueurs qui sont choisis parmi des chélates métalliques luminescents ou des groupes fluorescents.

4. Conjugué selon l'une quelconque des revendications 1 à 3, **caractérisé en ce qu'**il contient des groupes de liaison aux phases solides qui sont choisis parmi la biotine et les analogues de biotine.

5. Conjugué selon l'une quelconque des revendications 1 à 3, **caractérisé en ce que** le support polymère contient au moins un porteur de charge positive ou/et négative.

6. Conjugué selon la revendication 5, **caractérisé en ce que** le support polymère contient des groupes carboxylate comme porteurs de charge négative.

7. Conjugué selon l'une quelconque des revendications précédentes, **caractérisé en ce que** l'haptène est une molécule immunologiquement réactive ayant une masse moléculaire de 100-2000 Da, choisie parmi les principes actifs pharmacologiques, les hormones, les métabolites, les vitamines, les médiateurs et les neurotransmetteurs.

8. Conjugué selon l'une quelconque des revendications précédentes, **caractérisé en ce que** les unités monomères du support comprennent des acides aminés artificiels.

9. Conjugué selon la revendication 8, **caractérisé en ce que** les acides aminés artificiels sont choisis parmi la β-alanine, l'acide γ-aminobutyrique, l'acide ε-aminocaproïque, la norleucine et l'ornithine.

10. Procédé de préparation de conjugués comprenant un support polymère ayant au maximum 100 unités monomères, qui comprend, 1-10 molécules d'haptène et 1-10 groupes marqueurs ou de liaison aux phases solides couplés à des groupes réactifs, les unités monomères étant choisies parmi les acides aminés, les nucléotides, les analogues de nucléotides et les acides nucléiques peptidiques, **caractérisé en ce que** l'on synthétise un support polymère d'unités monomères sur une phase solide,
(a) en introduisant lors de la synthèse, en des positions prédéterminées du support, des dérivés monomères, qui sont couplés de manière covalente à des molécules d'haptène ou/et des groupes marqueurs ou de liaison aux phases solides, ou/et
(b) en couplant à des groupes réactifs du support, après la synthèse, des molécules d'haptène ou/et des groupes marqueurs ou de liaison aux phases solides activés,
le couplage aux groupes amine primaire ou/et thiol des monomères d'acides aminés dans la variante (b) étant réalisé après séparation des groupes protecteurs des dérivés monomères utilisés pour la synthèse des phases solides.

11. Procédé selon la revendication 10, **caractérisé en ce que** l'on synthétise un support peptide à partir de dérivés d'acides aminés.

12. Procédé selon la revendication 10 ou 11, **caractérisé en ce que**, dans la variante (a), les molécules d'haptène ou/et les groupes marqueurs ou de liaison aux phases solides sont couplés respectivement à un groupe amine primaire ou un groupe thiol du dérivé monomère.

13. Procédé selon l'une quelconque des revendications 10 à 12, **caractérisé en ce que** l'on couple, après la synthèse dans la variante (b), les molécules d'haptène et les groupes marqueurs ou de liaison aux phases solides aux groupes latéraux amine primaire du support, un dérivé monomère ayant un premier groupe protecteur pour le groupe latéral amine étant utilisé aux positions du support où un couplage avec des molécules d'haptène doit être réalisé et un dérivé monomère ayant un deuxième groupe protecteur pour le groupe latéral amine étant utilisé aux positions du support où un couplage avec des groupes marqueurs ou de liaison aux phases solides doit être réalisé et le premier et deuxième groupe protecteur étant choisis de sorte qu'une séparation sélective des groupes protecteurs soit possible.

14. Procédé selon la revendication 13, **caractérisé en ce que** le premier et le deuxième groupe protecteur sont choisis parmi les groupes protecteurs sensibles aux acides ou résistants aux acides.

15. Utilisation de conjugués selon l'une quelconque des revendications 1 à 9, comme antigène dans un procédé immunologique ou pour le diagnostic d'acides nucléiques.

16. Utilisation selon la revendication 15, **caractérisée en ce que** l'on utilise des conjugués qui contiennent plus de 1 molécule d'haptène, comme polyhaptène dans le procédé de détection immunologique.

17. Utilisation selon la revendication 15 ou 16 dans une immunoanalyse compétitive.

18. Utilisation selon la revendication 15 ou 16 dans une immunoanalyse pour la détection d'anticorps spécifiques.

19. Procédé de détection d'un analyte dans un liquide échantillon d'après le principe de l'immunoanalyse compétitive au format "analogue marqué", **caractérisé en ce que**
(a) l'on incube le liquide échantillon en présence d'une phase solide réactive avec un conjugué selon l'une quelconque des revendications 1 à 9, qui comprend un groupe marqueur, et un récepteur, qui est lié à la phase solide ou capable de liaison à une phase solide et peut engager une réaction immunologique spécifique avec l'analyte et le composant haptène du conjugué,
(b) l'on sépare éventuellement la phase solide du liquide d'incubation et
(c) l'on détermine la présence et/ou la quantité de l'analyte dans le liquide échantillon par mesure du composant marqueur du conjugué dans la phase solide et/ou dans le liquide d'incubation.

20. Procédé selon la revendication 19, **caractérisé en ce que** l'on utilise comme récepteur un anticorps biotinylé ou un fragment d'anticorps biotinylé et une phase solide revêtue de streptavidine ou d'avidine.

21. Procédé de détection d'un analyte dans un liquide échantillon d'après le principe de l'immunoanalyse compétitive au format "anticorps marqué", **caractérisé en ce que**
(a) l'on incube le liquide échantillon en présence d'une phase solide réactive avec un conjugué selon l'une quelconque des revendications 1 à 9, qui comprend un groupe de liaison aux phases solides, et un récepteur, qui porte un groupe marqueur et peut engager une réaction immunologique spécifique avec l'analyte et le composant haptène du conjugué,
(b) l'on sépare éventuellement la phase solide du liquide d'incubation et
(c) l'on détermine la présence et/ou la quantité de l'analyte dans le liquide échantillon par mesure du composant marqueur du récepteur dans la phase solide et/ou dans le liquide d'incubation.

22. Procédé selon la revendication 21, **caractérisé en ce que** l'on utilise un conjugué biotinylé et une phase solide revêtue de streptavidine ou d'avidine.

23. Procédé de détection d'un anticorps spécifique dans un liquide échantillon, **caractérisé en ce que** l'on
(a) incube le liquide échantillon avec au moins un conjugué selon l'une des revendications 1 à 9, qui est dirigé contre l'anticorps à détecter et
(b) détecte l'anticorps par liaison au conjugué.

24. Procédé selon la revendication 23, **caractérisé en ce que** l'on
(a) incube le liquide échantillon en présence d'une phase solide réactive et deux antigènes dirigés contre l'anticorps à détecter, le premier antigène portant un groupe marqueur et le deuxième antigène étant lié à la phase solide ou se présentant sous une forme capable de se lier à la phase solide,
(b) sépare le cas échéant la phase solide du liquide d'incubation, et
(c) détermine la présence ou/et la quantité de l'anticorps par détermination du marquage dans la phase solide ou/et dans la phase liquide,
un conjugué selon l'une quelconque des revendications 1 à 9 étant utilisé comme premier et/ou deuxième antigène.

25. Procédé selon la revendication 24, **caractérisé en ce que** l'on utilise comme premier antigène un conjugué marqué par un chélate métallique luminescent ou un groupe fluorescent.

26. Procédé selon la revendication 24 ou 25, **caractérisé en ce que** l'on utilise comme deuxième antigène un conjugué biotinylé et une phase solide revêtue de streptavidine ou d'avidine.
